Europäisches Patentamt

(19) European Patent Office

Office européen des brevets

(11) Numéro de publication: **0 246 979**
**B1**

(12) FASCICULE DE BREVET EUROPEEN

(45) Date de publication du fascicule du brevet:
24.10.90

(21) Numéro de dépôt: 87401150.5

(22) Date de dépôt: **21.05.87**

(51) Int. Cl.⁵: **G01N 1/10,** G01N 1/00,
C12M 1/26

(54) **Dispositif permettant de conserver la stérilité d'un milieu de culture en enceinte pendant le prélèvement d'échantillons.**

(30) Priorité: **22.05.86 FR 8607329**

(43) Date de publication de la demande:
**25.11.87 Bulletin 87/48**

(45) Mention de la délivrance du brevet:
**24.10.90 Bulletin 90/43**

(84) Etats contractants désignés:
**AT BE CH DE ES FR GB GR IT LI LU NL SE**

(56) Documents cités:
**DE-A- 3 338 782**
**FR-A- 2 250 109**
**FR-A- 2 524 144**
**US-A- 3 746 217**

(73) Titulaire: **CENTRE NATIONAL DE LA RECHERCHE SCIENTIFIQUE (CNRS), 15, Quai Anatole France, F-75007 Paris(FR)**

(72) Inventeur: **Romette, Jean-Louis, Chemin de Cambronne, F-60129 Orrouy(FR)**
Inventeur: **Fourreau, Joel Gérald, 17, rue Winston Churchill Les Amandiers, Apt. 15, F-60200 Complègne(FR)**

(74) Mandataire: **Phélip, Bruno et al, c/o Cabinet Harlé & Phélip 21, rue de La Rochefoucauld, F-75009 Paris(FR)**

## Description

La présente invention est du domaine des prélèvements d'échantillons en enceinte stérile. Elle utilise plus particulièrement un dispositif permettant de conserver la stérilité d'un milieu de culture en enceinte pendant le prélèvement d'échantillons destinés à l'analyse.

Jusqu'à présent, les dispositifs de la technique antérieure, qui étaient la plupart actionnés manuellement, ne permettaient pas de prélever des échantillons sans compromettre la stérilité du milieu de culture. Du fait que l'utilisateur devait faire son prélèvement manuellement, avec les multiples inconvénients que cela comporte, il était difficile de prélever des échantillons de volume difficilement contrôlable dont on ne pouvait en outre garantir l'absence de contamination par le milieu extérieur. Toutefois, un des risques les plus importants des dispositifs de la technique antérieure était celui de contaminer le contenu du fermenteur.

Un des objets de la présente invention est de fournir un dispositif de prélèvement avec lequel on peut effectuer de façon reproductible, un prélèvement de volume connu.

Un autre objet de la présente invention est de fournir un dispositif de prélèvement dans lequel le prélèvement de volume connu est effectué en ligne, tout en assurant la non-contamination du milieu de culture dans lequel est effectué ledit prélèvement.

Encore un autre objet de l'invention, est de réaliser un dispositif de prélèvement assurant la diminution des risques de contamination de la fermentation grâce à la possibilité de réaliser une stérilisation aisée des pièces du dispositif impliquées dans le prélèvement d'un échantillonde volume rigoureusement constant, tout en permettant une dilution automatique de l'échantillon prélevé au moyen d'un solvant ainsi que le refroidissement des éléments mobiles du dispositif et leur séchage au moyen d'un fluide stérile.

La présente invention a donc pour objet un dispositif permettant de conserver la stérilité d'un milieu de culture en enceinte pendant le prélèvement d'échantillons destinés à l'analyse, ce dispositif étant caractérisé en ce qu'il comporte, à l'intérieur d'un bâti, une première chambre, dite de prélèvement, dans laquelle coulisse un piston pourvu à une extrémité de moyens permettant le prélèvement d'un échantillon à analyser et une seconde chambre, dite de dilution, reliée à la première chambre par un passage, cette seconde chambre étant divisée en deux zones par des moyens de butée contre lesquels viennent en aboutement au moins deux pistons coulissants respectivement de part et d'autre la première chambre présentant au moins un orifice de sortie pour l'échantillon prélevé, tandis que la seconde chambre comporte au moins un orifice d'admission pour un solvant.

Le dispositif de prélèvement selon des modifications la présente invention est en outre remarquable par les points suivants:

- on a prévu des moyens d'étanchéité entre les pistons et les chambres correspondantes;

- le piston de la première chambre présente dans sa zone opposée à la zone de prélèvement un épaulement;

- on a prévu dans la seconde chambre un butoir dont le volume et choisi de façon à déterminer le volume de dilution limité par les faces en regard des pistons et les faces internes de ladite chambre;

- le butoir est mobile et actionné par des moyens extérieurs, par exemple en moteur pas à pas commandé par des moyens électro-informatiques permettant ainsi de régler le volume de dilution, donc le coefficient de dilution de l'échantillon;

- les divers pistons sont actionnés par des moyens moteurs, par exemple, du type hydraulique, pneumatique ou magnétique. Dans le cas d'une commande pneumatique des pistons, on utilise avantageusement une source d'air comprimé, une pluralité de vannes distributrices étant prévues et commandées par des moyens mécaniques, par exemple du type automate à cames, ou bien par des moyens électro-informatiques, par exemple un micro-processeur.

Selon une autre variante de réalisation du dispositif de prélèvement selon la présente invention, ce dernier comporte deux corps solidarisés l'un à l'autre, par exemple par vissage, définissant une première chambre cylindrique dite de prélèvement et une seconde chambre cylindrique dite de dilution, dans lesquelles se déplacent respectivement un et trois pistons baladeurs. Cette variante de réalisation s'utilise avantageusement en disposant la chambre de prélèvement en position supérieure par rapport à la chambre de dilution.

L'ensemble de ces modifications a pour but: d'assurer une meilleure stérilité du piston préleveur lorsqu'il pénètre dans le fermenteur, de garantir une bonne stérilisation; d'assurer une séparation entre les condensats de stérilisation et le liquide prélevé; de garantir une bonne exactitude de dilution; de diminuer les coûts des pièces en simplifiant l'usinage; et d'obtenir une stérilisation plus rapide et donc un échauffement global moindre.

Dans tous les cas, on a également prévu des moyens d'admission de solvant et des moyens permettant le balayage-nettoyage du dispositif de prélèvement, après chaque cycle de prélèvement.

D'autres avantages de l'invention apparaîtront à la lecture de la description suivante, d'une forme de réalisation non limitative de dispositif de prélèvement, en référence aux dessins annexés, dans lesquels:

Fig. 1 est une vue en coupe longitudinale axiale d'un dispositif de prélèvement ou échantillonneur selon l'invention, monté sur un piquage d'une enceinte de culture ou fermenteur;

Fig. 2 est une vue plus en détail d'une coupe longitudinale axiale d'un échantillonneur selon l'invention, pendant la séquence de stérilisation;

Fig. 3 est une vue analogue à la Figure 1 lors de la séquence de prélèvement de l'échantillon;

Fig. 4 est une vue analogue aux Figures précédentes, lors de la séquence où l'échantillon est ramené dans l'échantillonneur;

Fig. 5 est une vue analogue aux Figures précédentes, pendant la séquence de préparation du volume d'eau destiné à la dilution;

Fig. 6 est une vue analogue aux Figures précédentes pendant la séquence de dilution-rinçage;

Fig. 7 est une vue en coupe longitudinale axiale d'une variante de réalisation du dispositif échantillonneur de l'invention, pendant la séquence de stérilisation;

Fig. 8 est une vue analogue à la Figure 1 pendant la séquence de fin de stérilisation-pré-prélèvement;

Fig. 9 est une vue analogue à la Figure 1 pendant la séquence de prélèvement;

Fig. 10 est une vue analogue à la Figure 1 pendant la séquence de fermeture d'admission de vapeur et fin de passage;

Figs 11A et 11B sont respectivement des vues analogues à la Figure 1 pendant la séquence de dilution du liquide prélevé;

Fig. 12 est une vue analogue à la Figure 1 de la séquence de fin de dilution.

Figs 13A et 13B sont respectivement des vues analogues à la Figure 1 pendant la séquence et remise en position des pièces pour un nouveau cycle de stérilisation.

Fig. 14 est un schéma d'ensemble du dispositif de prélèvement d'échantillon, selon l'invention.

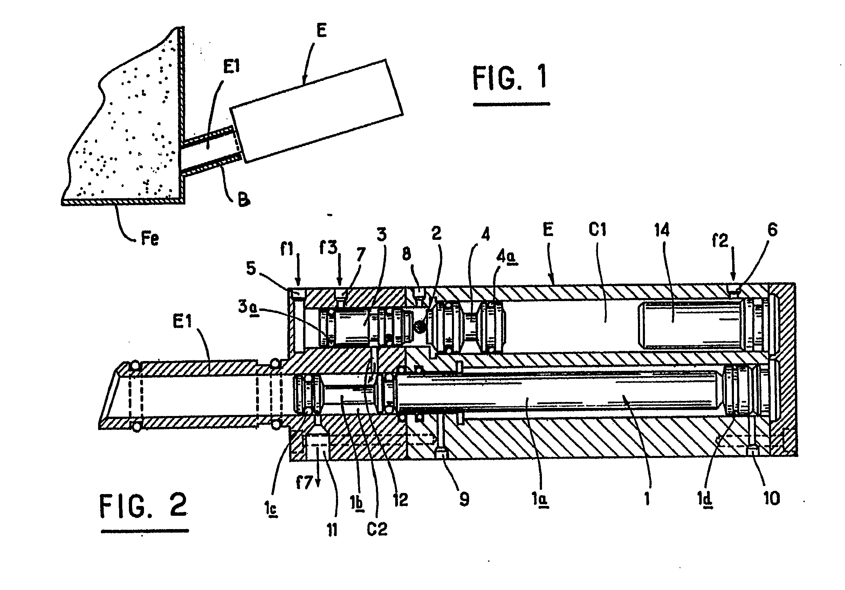

Comme représenté sur la Figure 1, l'échantillonneur E est monté dans un piquage B d'un fermenteur Fe par l'intermédiaire d'un embout E1. On notera que le piquage B est dirigé obliquement vers le haut, par rapport à la paroi du fermenteur, cette inclinaison étant destinée à renvoyer le condensat vers l'intérieur du fermenteur.

Comme représenté plus en détail sur les Figures 2 à 6, l'échantillonneur selon la présente invention est constitué d'un bati E dans lequel on a aménagé une chambre supérieure C1 et une chambre inférieure C2 se prolongeant par l'embout E1. Dans la chambre inférieure C2, dite de prélèvement, est logé un piston 1 présentant dans sa zone d'extrémité opposée à l'embout E1 un épaulement 1$\underline{a}$. A son autre extrémité, le piston est conformé de façon à définir un évidement torique 1$\underline{b}$. Des moyens d'étanchéité constitués par des joints toriques 1$\underline{c}$ sont prévus de chaque côté de la chambre torique 1$\underline{b}$. De même des moyens d'étanchéité sont également prévus au voisinage de la zone de l'épaulement 1$\underline{a}$, ces moyens d'étanchéité étant également constitués par des bagues toriques 1$\underline{d}$. Dans la chambre supérieure C1 ou de dilution, sont montés de part et d'autre d'une butée centrale 2, deux pistons 3 et 4 pourvus respectivement dans leur zone d'extrémité de bagues toriques d'étanchéité 3$\underline{a}$, 4$\underline{a}$. La chambre de dilution C1 est pourvue d'orifices d'admission d'air, 5, 6, selon les flèches f1, f$\underline{2}$, d'un orifice d'admission de vapeur 7 selon la flèche f$\underline{3}$ et d'un orifice d'admission de solvant de dilution 8.

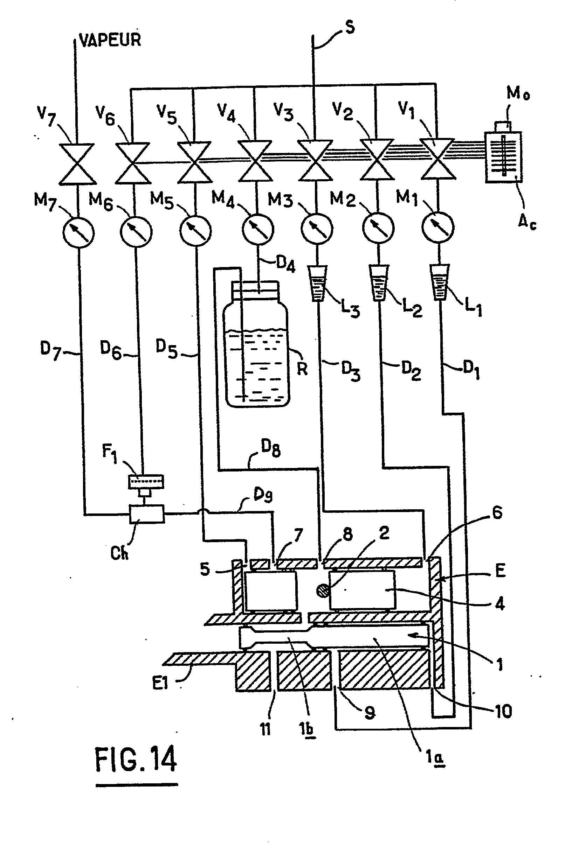

La chambre C2 ou de prélèvement est pourvue de deux orifices d'admission-sortie d'air 9, 10, selon les flèches f$\underline{8}$, f4, f$\underline{5}$, f$\underline{6}$, respectivement. La chambre C2 est également pourvue d'un orifice de purge 11 selon la flèche f$\underline{7}$. Les chambres C1 et C2 sont réunies par un passage ou orifice 12. Dans le schéma général de l'échantillonneur, selon l'invention telle que représenté sur la Figure 14, on voit que l'échantillonneur fonctionne avec une source d'air comprimé S alimentant par une série de vannes V1, V2, V3, V5 les orifices respectifs 9, 10, 6, 5 par les conduits respectifs D1, D2, D3, D5. L'air comprimé est également admis, d'une part, par la vanne V4 et le conduit D4 à un récipient R renfermant un solvant approprié, par exemple de l'eau, un récipient dans lequel l'eau soumise à la pression d'air comprimé passe par le conduit D8 à l'orifice d'admission de solvant 8, et d'autre part, par la vanne V6 et le conduit D6 à un filtre F$_1$ où l'air étant débarrassé de ses microorganismes et impuretés est transféré à une chambre de mélange Ch où cet air purifié vient se mélanger avec de la vapeur dont l'admission est commandée par une vanne V7 via un conduit D7. Le mélange air-comprimé/vapeur est ensuite amené par un conduit D9 à l'orifice d'admission de vapeur 8. Les vannes V1 à V7 sont avantageusement des électrovannes commandées par un automate à came Ac, lui-même actionné par un motoréducteur Mo et associées à des manodétendeurs M1 à M7. En aval des manodétendeurs M1, M2, M3, on peut prévoir des lubrificateurs L1, L2, L3, cette mesure n'est toutefois pas obligatoire car l'échantillonneur selon l'invention peut également fonctionner de façon performante, sans l'aide d'agent de lubrification.

Le cycle de fonctionnement de l'échantillonneur selon la présente invention est le suivant:

- lorsque l'échantillonneur E est implanté sur le fermenteur F, comme représenté sur la Figure 1, on peut décomposer son fonctionnement en cinq opérations principales comme représentées respectivement sur les Figures 2 à 6.

- comme représenté sur la Figure 2, la première opération consiste, avant utilisation de l'échantillonneur, à stériliser les éléments constitutifs contaminés par le milieu extérieur, à cet effet, les vannes V3, V5, V6 et V7 sont ouvertes, les autres vannes étant fermées, de façon à, d'une part, faire venir les pistons 3 et 4 en aboutement contre la butée 2, sous l'effet de l'air admis selon f1 et f2, dans la chambre C1 tandis que le mélange d'air filtré provenant du filtre F$_1$ et la vapeur soient mélangés dans la chambre Ch avant d'être admis dans l'intérieur de l'échantillonneur par l'orifice d'admission 7. Le mélange air/vapeur de purge sort alors par l'orifice 11 selon la flèche f$\underline{7}$.

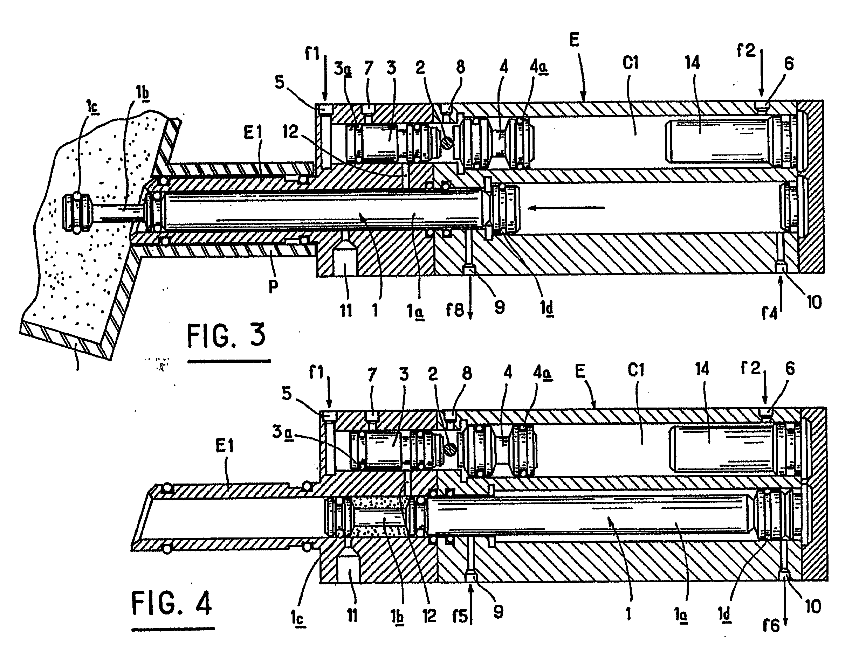

La seconde opération, telle qu'illustrée sur la Figure 3, consiste à prélever l'échantillon. A cet effet, les vannes V3 et V5 restent à l'état ouvert, tandis que l'on ouvre la vanne V2 de façon à amener la chambre de prélèvement 1$\underline{b}$ du piston 1 à l'intérieur du fermenteur. Dans cette séquence, les autres vannes V1, V4, V6 et V7 restent à l'état fermé.

La troisième opération, comme représentée sur la Figure 4, consiste à ramener l'échantillon à l'intérieur de l'échantillonneur, et ce, en fermant la vanne V2 et en ouvrant la vanne V1, les vannes V3 et V5 restant toujours fermées, de même que les autres vannes. L'air admis selon f5 par l'orifice 9 agit sur l'épaulement 1$\underline{a}$ du piston 1, pour ramener ce dernier en aboutement contre la paroi de la chambre C1; cet air s'écoulant selon f$\underline{6}$ par l'orifice 10.

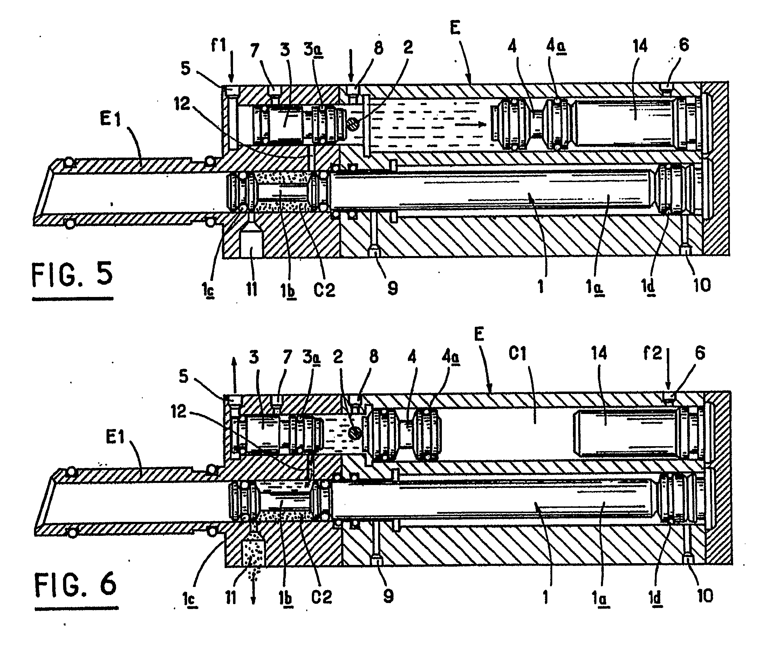

La quatrième opération représentée par la Figure 5 consiste à préparer un volume de solvant, par exemple de l'eau, destiné à la dilution de l'échantillon déjà prélevé selon les opérations précédentes. A cet effet, toutes les vannes du dispositif sont à l'état fermé, à l'exception de la vanne V4 et de la vanne V5. La pression d'air comprimé issue de cette dernière amène le piston 3 en aboutement contre la butée 2, tandis que la pression d'air issue de la vanne V4, provoque, comme décrit ci-dessus, une arrivée de solvant, par exemple de l'eau à l'intérieur de la chambre C2 par l'orifice d'admission 8. On notera que les faces en regard des pistons 3 et 4 définissent avec les faces internes de la chambre C1 un volume de dilution devenant maximal lorsque le piston 4 vient en aboutement contre le butoir 14 de la paroi de fond de ladite chambre C1.

La cinquième opération dite de "dilution-rinçage" consiste à entraîner vers l'analyseur l'échantillon dûment dilué, et ce, par ouverture de la vanne V3 qui repousse le piston 4 en aboutement contre la butée 2, tandis que le piston 3 vient en aboutement contre la paroi opposée de la chambre C1, libérant ainsi le passage 12 entre les chambres C1 et C2 dans lequel s'écoule le liquide de dilution-solvant, qui se combinant dans la chambre torique 1b s'écoule vers l'analyseur, non représenté, sur les dessins, par l'orifice d'évacuation ou de purge 11.

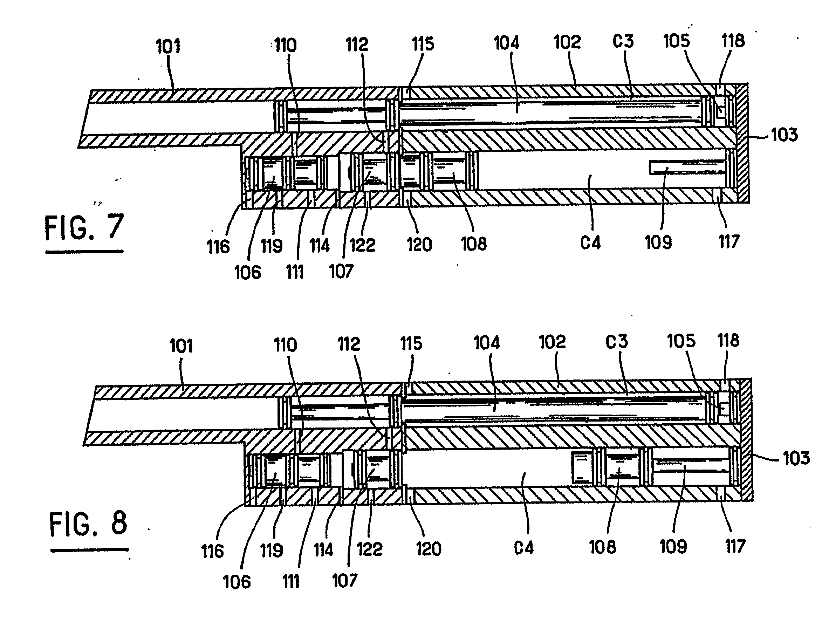

Dans la variante de réalisation représentée sur les figures 7 à 13, le dispositif échantillonneur stérile selon l'invention est constitué de deux corps 101 et 102 reliés entre eux par des vis. Le corps 101 fait office d'embout et est fixé sur le fermenteur. A l'extrémité libre du corps 102 est prévue une plaque d'obturation 103. L'assemblage des trois éléments 101, 102, 103 détermine deux chambres cylindriques, à savoir une chambre supérieure ou de prélèvement C3 et une chambre inférieure ou de dilution C4. Dans la chambre supérieure C3 est monté un piston préleveur 104 susceptible de venir en aboutement contre un bouchon d'extrémité 105 qui assure l'étanchéité de la chambre C3. A l'intérieur de la chambre C4 sont montés respectivement trois pistons baladeurs, à savoir un piston dit de condensat 106, un piston dit de vapeur 107 et un piston dit de dilueur 108, ce dernier piston étant suceptible de venir en aboutement contre un bouchon d'extrémité 109 destiné à assurer l'étanchéité de la chambre C4.

Cette variante de réalisation d'échantillonneur stérile fonctionne de la manière suivante:

Dans une première séquence de fonctionnement, le piston 104 est en butée à droite contre le bouchon d'étanchéité 105. Le piston 106 est poussé en butée, à gauche de la chambre C4, libérant ainsi le passage par les alésages 110, 111. Le piston 107 est poussé en butée à droite de la zone de la chambre C3, sur l'épaulement de la chambre C3 libérant ainsi le passage par les alésages 112, 122, tandis que le piston 108 vient en butée contre le piston 107. Ces positions sont obtenues sous l'effet de la pression d'air comprimé appliqué par les orifices 114 et 115. La pression en 114 est relâchée avant la fin de la stérilisation par mise à pression atmosphérique pour éviter qu'une légère fuite du joint de la chambre de prélèvement C3 ne vienne recontaminer celle-ci à l'arrêt de la vapeur. Les alésages 116, 117 et 118 sont à pression atmosphérique, tandis que les électrovannes associées aux alésages 119 et 120 sont fermées. La vapeur est admise par l'orifice 122, elle remonte le long du corps du piston 107, traverse l'alésage 112 puis la chambre de prélèvement du piston 104 et s'élimine, ainsi que les condensats, selon la pente naturelle de prélèvement par l'alésage 110, puis le long du corps du piston 106 par l'alésage 111. Après soufflage la pressurisation de la chambre pour sa montée en température est obtenue par séquencement sur une électrovanne branchée sur l'alésage 111. Les diamètres des alésages et des corps de piston sont étudiés pour permettre un passage aisé de la vapeur. Pour la stérilisation de la chambre de prélèvement, le positionnement correct du piston 104 est assuré par l'excitation d'un capteur de butée, non représenté aux dessins, contre le bouchon d'étanchéité 105. Dans la seconde séquence de fonctionnement représentée sur la Figure 8, on procède, avant la fin de la stérilisation, au remplissage de la chambre à eau de la chambre de prélèvement C3 par une admission à travers l'alésage 120, en considérant que l'alésage 117 reste sous pression atmosphérique depuis la séquence de fonctionnement représentée sur la Figure 7. Le piston 108 vient alors en butée contre le bouchon d'étanchéité 109, tandis que le piston 107 n'est pas déplacé puisqu'il est maintenu par la pression d'air comprimé provenant de l'alésage 114.

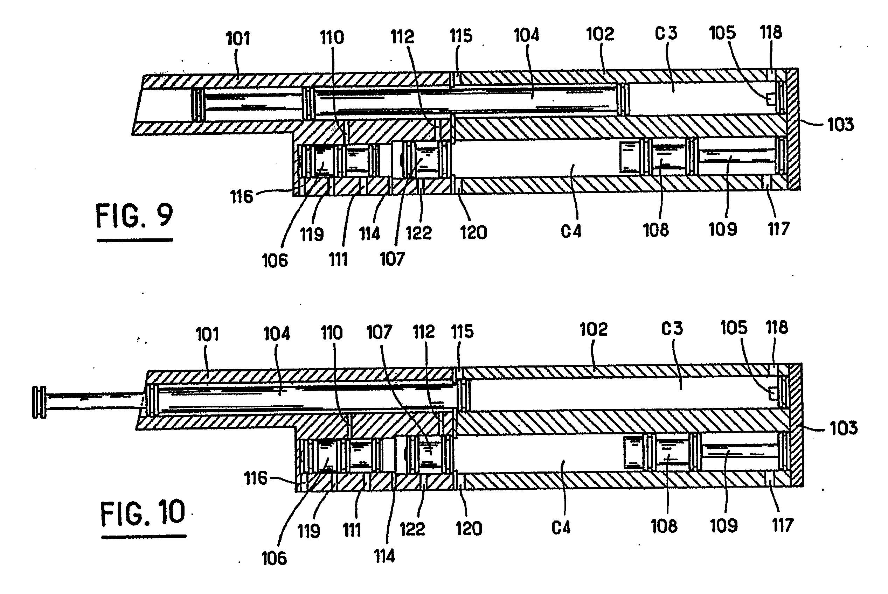

Dans une troisième séquence de fonctionnement, lorsque le temps de stérilisation de la chambre est écoulé, le système passe en position "prélèvement". Après la fermeture de l'électrovanne associée à l'alésage 115, l'électrovanne susceptible d'occuper trois positions, à savoir: admission de l'air comprimé, purge, arrêt, de l'air comprimé est admis par l'alésage 118. Lors de son avancée, le piston 104 libère l'alésage 112 qui permet le remplissage en mélange vapeur/air de la chambre de la tige du piston 104 (sans évacuation) puis libère l'alésage 110 qui permet l'établissement d'un circuit de vapeur par les alésages 122, 112, 110, 111 qui stérilisent la tige du piston 104 ainsi que la chambre où elle se déplace. Selon la séquence de fonctionnement 4 représentée sur la Figure 9, lorsque le piston 104 arrive en fin de course gauche, en butée sur l'épaulement de la chambre C3, il est possible d'arrêter le passage de la vapeur. L'électrovanne admettant celle-ci à l'orifice 122 est alors fermée après celle de purge à l'orifice 111.

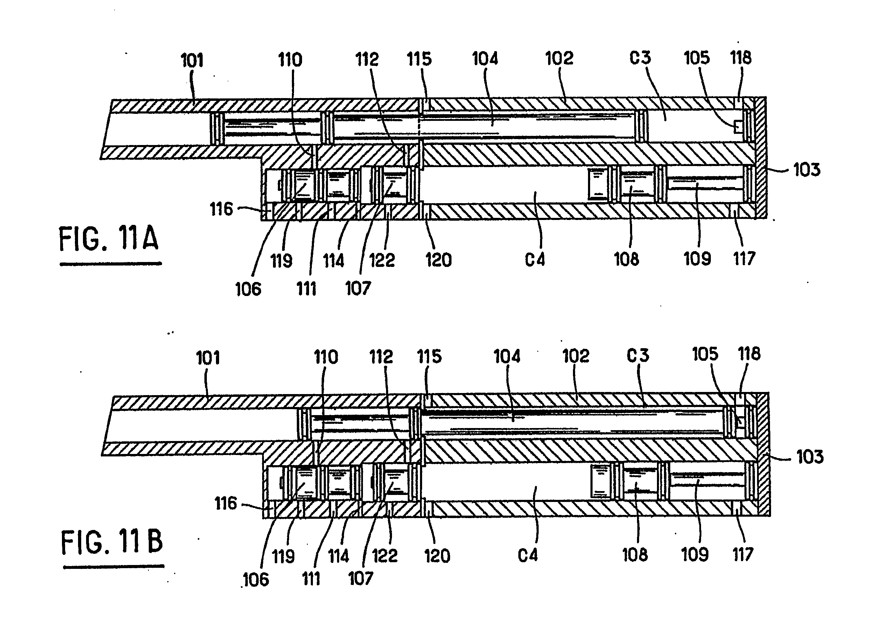

Dans la séquence de fonctionnement représentée sur les Figures 11A et 11B, l'alésage 118 est alors mis à pression atmosphérique et de l'air comprimé est admis par l'alésage 115 provoquant le retrait du piston 104 et la mise en pression d'air comprimé des chambres respectives des pistons 106 et 107.

Le piston 104 se déplace donc vers la droite. Dès que la chambre de prélèvement débouche dans l'alésage 110, ce qui est détecté par un capteur de position, non représenté aux dessins, du piston 104, la vanne de commande de l'orifice 114 se met à la pression atmosphérique et de l'air comprimé est envoyé par l'orifice 116, provoquant le déplacement du piston 106 sur la droite jusqu'à venir en butée, comme cela est plus particulièrement représenté sur la Figure 11A. Les alésages 110 et 119 communiquent alors, la pression 119 étant la pression atmosphérique. Le piston 104 peut alors continuer sa course sur la droite jusqu'à rentrer en butée avec le bouchon d'étanchéité 105, comme cela est plus particulièrement représenté sur la Figure 11B. La pression résiduelle dans l'espace annulaire du piston 106 commence à chasser le liquide selon le trajet passant par les alésages 112, 110, 119. Il convient que le piston 104 marque une pause au moment où la chambre de prélèvement rentre en communication avec l'orifice 110, cette pause étant utilisée pour faire déplacer le piston 106 de façon à éviter de voir le piston 104 atteindre sa position droite en butée contre le bouchon 105 alors que le piston 106 est toujours à gauche et le piston 107 toujours à droite. Un éventuel déséquilibre de pression entre les espaces annulaires respectifs des pistons 106 et 107 pourrait alors faire refluer le liquide de la chambre de prélèvement au travers de l'orifice 112 vers l'alésage 122.

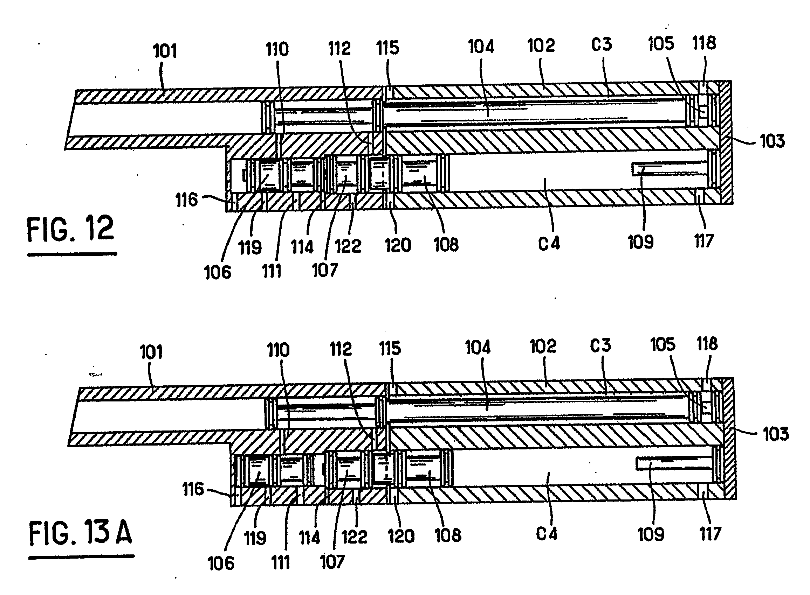

Dans la séquence de fonctionnement représenté sur la Figure 12, de l'air comprimé est alors envoyé par l'alésage 117, alors que la pression est toujours maintenue en 116, provoquant, sous la poussée du piston 108, une avancée de la colonne liquide qui déplace le piston 107 et libère l'alésage 112, le piston 107 venant alors en butée contre le piston 106. Le liquide passe donc dans la chambre de prélèvement, reflue par l'alésage 110 et s'échappe par l'alésage 119. L'extrémité du piston 108 vient alors s'emboîter dans la zone d'épaulement de la chambre C4.

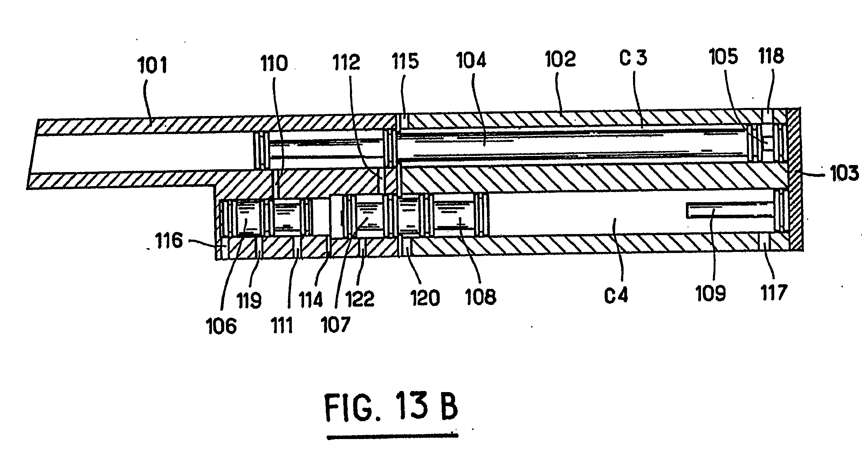

Dans la séquence de fonctionnement représentée sur les Figures 13A et 13B, il est possible de commencer un nouveau cycle de stérilisation, étant donné que le cycle de dilution est terminé. Pour ce faire, l'alésage 116 est mis à la pression atmosphérique. La mise en pression de l'alésage 114 provoque le positionnement du piston 106 en position de stérilisation, le trajet par les alésages 110 et 111 étant rendu libre. Le piston 107 est maintenu par le piston 108 comme représenté plus spécialement sur la Figure 13A. L'alésage 117 est alors mis sous pression atmosphérique. Le piston 107 repousse le piston 108 et libère le trajet passant par les alésages 122 et 112 d'arrivée de vapeur. Il est important que l'alésage 119 soit alors condamné avant que le passage passant par les alésages 122, 112 soit ouvert, de façon à éliminer le risque d'introduction de vapeur dans le prélèvement, ladite vapeur étant alors admise par la vanne associée à l'alésage 122, comme cela est plus particulièrement représenté sur la Figure 13B. L'opérateur se retrouve alors dans la situation lui permettant de réaliser la séquence 1.

L'échantillonneur selon la présente invention permet le contrôle de la non-contamination d'un milieu de culture en réalisant des cycles d'utilisation réelle pendant plusieurs jours. Selon un mode de réalisation préféré, on peut par exemple opérer sur un fermenteur de 20 litres, placé dans les conditions de culture normale, à l'exception notable de toute intervention pouvant induire une contamination et, bien entendu, sans innoculation. Après fermentation normale, on pourra, en utilisant l'échantillonneur selon l'invention, alimenter un analyseur, par exemple un appareil utilisant le principe des électrodes à enzyme, pour effectuer rapidement des dosages de molécules organiques en milieu liquide. Tout appareil, qui par sa conception nécessite qu'on lui fournisse des échantillons de volume constant, est rendu particulièrement performant grâce à son association avec l'échantillonneur selon la présente invention. On notera que la présente invention n'a été rendue possible que par la collaboration active entre le laboratoire de technologie enzymatique de l'Université Technologique de Compiègne, laboratoire associé au Centre National de la Recherche Scientifique N.338, et les inventeurs. Ainsi se trouve résolu, selon la présente invention, le problème de l'échantillonnage automatique respectant le milieu dans lequel l'échantillon est prélevé et ce, en obtenant des volumes constants à partir desquels on peut réaliser les différentes opérations d'analyse désirées.

Il est clair que la présente invention n'est nullement limitée à la forme de réalisation décrite ci-dessus en référence aux dessins annexés, mais qu'elle englobe toutes les modifications et variantes à la portée de l'homme de l'art. C'est ainsi que l'on peut modifier à volonté la forme des pistons, les rendre télescopiques ou à géométrie variable, tout en respectant les conditions d'étanchéité nécessaires à l'intérieur des chambres. De même, si l'on a utilisé des moyens pneumatiques, il n'en reste pas moins que n'importe quel moyen d'actionnement peut être utilisé à condition toutefois, de respecter les conditions d'étanchéité et de stérilité nécessaires au bon fonctionnement du dispositif de l'invention.

## Revendications

1. Dispositif permettant de conserver la stérilité d'un milieu de culture en enceinte pendant le prélèvement d'échantillons destinés à l'analyse, comportant, ménagé dans un bâti (E; 101–103), une première chambre (C2; C3) dite en prélèvement, dans laquelle est monté coulissant un piston (1; 104) pourvu d'une extrémité de moyens (1b) permettant le prélèvement d'un échantillon à analyser, et une seconde chambre (C1; 104), dite de dilution reliée à la première chambre par un passage (12; 112), cette seconde chambre étant divisée en deux zones par des moyens de butée (2) contre lesquels peuvent venir en aboutement au moins deux pistons (3, 4; 106–108) coulissant respectivement de part et d'autre de la butée (2), la première chambre présentant au moins un orifice de sortie pour l'échantillon prélevé, la seconde chambre (C1; C4) comportant au moins un orifice d'admission (8; 120) pour un solvant de dilution.

2. Dispositif selon la revendication 1, caractérisé en ce que le piston (1) présente dans la zone opposée aux moyens de prélèvements un épaulement (1a).

3. Dispositif selon l'une des revendications 1 et 2, caractérisé en ce que entre les pistons (3, 4;1) et les parois des chambres correspondantes (C1; C2) sont prévus des moyens d'étanchéité (3a, 4a; 1c, 1d), par exemple des bagues toriques.

4. Dispositif selon l'une quelconque des revendications 1 à 3, caractérisé en ce que les moyens permettant le prélèvement d'échantillons sont constitués par une chambre torique (1b) ménagée périphériquement sur le piston (1).

5. Dispositif selon l'une quelconque des revendications 1 à 4, caractérisé en ce que la chambre de dilution (C1) est pourvue d'un boutoir (14) de volume choisi de façon à déterminer le volume de dilution limité par les faces en regard des pistons (3, 4) et les faces internes de ladite chambre (C1).

6. Dispositif selon la revendication 5, caractérisé en ce que le butoir (14) est mobile et actionné par des moyens extérieurs — par exemple un moteur pas à pas, commandé par des moyens électro-informatiques — permettant ainsi de régler le volume de dilution, donc le coefficient de dilution de l'échantillon.

7. Dispositif selon l'une quelconque des revendications 1 à 6, caractérisé en ce que les pistons (1; 3, 4) sont actionnés par des moyens moteurs choisis parmi ceux du type hydraulique, pneumatique ou électromagnétique.

8. Dispositif selon l'une quelconque des revendications 1 à 7, caractérisé en ce que lorsque l'on utilise des moyens d'actionnement pneumatiques des pistons, par exemple, par l'intermédiaire d'une source d'air comprimé, des orifices d'admission-sortie d'air (5, 6; 11, 10) sont prévus respectivement dans les chambres (C1, C2).

9. Dispositif selon l'une quelconque des revendications 1 à 8, caractérisé en ce que la chambre de dilution (C1) comporte un orifice d'admission (7) destiné à d'admission d'un mélange vapeur/air stérile destiné au nettoyage-balayage de la chambre de dilution.

10. Dispositif selon l'une quelconque des revendications 1 à 9, caractérisé en ce que les moyens moteurs sont commandés par un automate à cames (Ac) destiné à la commande selon un cycle pré-établi des électrovannes d'admission (V1, V2, V3, V4, V5, V6, V7) d'admission des différents fluides dans le dispositif de prélèvement.

11. Dispositif selon l'une quelconque des revendications 1 à 9, caractérisé en ce que sont prévus des moyens électro-informatiques pour la commande selon le cycle désiré des vannes (V1, V2, V3, V4, V5, V6, V7).

12. Dispositif selon la revendication 1, caractérisé en ce qu'il comporte deux corps (101, 102) solidarisés l'un à l'autre et définissant une première chambre cylindrique supérieure, dite de prélèvement, (C3) et une seconde chambre cylindrique inférieure, dite de dilution (C4), la première chambre (C3) renfermant un piston (104) conformé pour présenter un espace périphérique de prélèvement tandis que la seconde chambre (C4) renferme trois pistons baladeurs (106, 107, 108).

13. Dispositif selon la revendication 12, caractérisé en ce que les corps (101, 102) sont raccordés à une extrémités par une plaque (103) formant appui pour des bouchons ou butées d'étanchéité (105, 109) fixés respectivement dans les chambres (C3, C4).

**Patentansprüche**

1. Vorrichtung zur Aufrechterhaltung der Sterilität eines Kulturmediums in einem abgeschlossenen Raum während der Entnahme von Proben, die für die Analyse bestimmt sind, umfassend in einem Gehäuse (E; 101–103), eine erste Kammer (C2; C3), die als Entnahmekammer bezeichnet wird und in der ein Kolben (1; 104) verschiebbar montiert ist, wobei dieser an einem Ende mit Mitteln (1b) ausgestattet ist, die die Entnahme von zu analysierenden Proben erlauben, sowie einer zweiten Kammer (C1; 104), die als Verdünnungskammer bezeichnet wird und die mit der ersten Kammer über einen Durchgang (12; 112) verbunden ist, wobei diese zweite Kammer durch Wiederlagervorrichtungen (2) in zwei Zonen aufgeteilt ist, auf welche wenigstens zwei Kolben (3, 4; 106–108) auftreffen können, wobei diese Kolben jeweils von der einen und der anderen Seite verschiebbar auf das Wiederlager (2) treffen, wobei die erste Kammer mit wenigstens einer Ausgangsöffnung für die entnommene Probe ausgestattet ist, und die zweite Kammer (C1; C4) wenigstens eine Einlaßöffnung (8; 120) für ein Lösungsmittel zur Verdünnung aufweist.

2. Vorrichtung nach Anspruch 1, dadurch gekennzeichnet, daß der Kolben (1) in der den Mitteln zur Entnahme entgegengesetzten Zone einen Vorsprung (1a) aufweist.

3. Vorrichtung nach einem der Ansprüche 1 und 2, dadurch gekennzeichnet, daß zwischen den Kolben (3, 4; 1) und den Wänden der entsprechenden Kammern (C1; C2) Mittel zur Abdichtung (3a, 4a; 1c, 1d), z.B. torische Ringe vorgesehen sind.

4. Vorrichtung nach irgendeinem der Ansprüche 1 bis 3, dadurch gekennzeichnet, daß die Mittel zur Probenentnahme aus einer torischen Kammer (1b) bestehen, die peripherisch um den Kolben (1) angeordnet ist.

5. Vorrichtung nach irgendeinem der Ansprüche 1 bis 4, dadurch gekennzeichnet, daß die Verdünnungskammer (C1) mit einem Anschlag (14) ausgestattet ist, dessen Volumen das Volumen der Verdünnung bestimmt, wobei dieses Volumen durch die gegenüberstehenden Seiten der Kolben (3, 4) und die inneren Seitenflächen der besagten Kammer (C1) begrenzt wird.

6. Vorrichtung nach Anspruch 5, dadurch gekennzeichnet, daß der Anschlag (14) beweglich ist und durch externe Mittel, z.B. einen Schrittmotor bewegt wird, der computergesteuert ist, und so die Regulierung des Volumens der Verdünnung und infolgedessen auch des Koeffizienten der Verdünnung der Probe erlaubt.

7. Vorrichtung nach irgendeinem der Ansprüche 1 bis 6, dadurch gekennzeichnet, daß die Kolben (1; 3, 4) mit Hilfe von Antriebsmitteln, ausgewählt aus solchen hydraulischer, pneumatischer oder elektromagnetischer Art, bewegt werden.

8. Vorrichtung nach irgendeinem der Ansprüche 1 bis 7, dadurch gekennzeichnet, daß bei Benutzung eines pneumatischen Antriebsmittels für die Kolben mittels einer Druckluftquelle, Lufteinlaß- und -auslaßöffnungen (5, 6; 11, 10) jeweils in den Kammern (C1, C2) vorgesehen sind.

9. Vorrichtung nach irgendeinem der Ansprüche 1 bis 8, dadurch gekennzeichnet, daß die Verdünnungskammer (C1) eine Einlaßöffnung (7) aufweist, die für den Einlaß sterilen Dampf/Luft-Gemisches zur Reinigung/Spülung der Verdünnungskammer bestimmt ist.

10. Vorrichtung nach irgendeinem der Ansprüche 1 bis 9, dadurch gekennzeichnet, dass die Antriebsmittel durch einen Nockenautomaten (Ac) gesteuert werden und der dafür bestimmt ist, den Einlaß der verschiedenen Flüssigkeiten in die Entnahmevorrichtung mit Hilfe von Einlaß-Elektroventilen (V1, V2, V3, V4, V5, V6, V7) nach einem vorgegebenen Zyklus zu steuern.

11. Vorrichtung nach irgendeinem der Ansprüche 1 bis 9, dadurch charakterisiert, daß Mittel der elektronischen Datenverarbeitung für die Steuerung der Schieber (V1, V2, V3, V4, V5, V6, V7) nach dem gewünschten Zyklus vorgesehen sind.

12. Vorrichtung nach Anspruch 1, dadurch gekennzeichnet, daß sie zwei kraftschlüssig miteinander verbundene Körper (101, 102) aufweist, wobei eine erste zylindrische obere Kammer, genannt Entnahmekammer (C3) und eine zweite zylindrische untere Kammer, genannt Verdünnungskammer (C4) gebildet wird, und die erste Kammer (C3), einen Kolben (104) enthält, der so ausgebildet ist, daß er einen Entnahme-Ringraum aufweist, während die zweite Kammer (C4) drei Schiebekolben (106, 107, 108) enthält.

13. Vorrichtung nach Anspruch 12, dadurch gekennzeichnet, daß die Räume (101, 102) an einem Ende durch eine Platte (103) in Verbindung stehen, die eine Stütze für die Dichtungszapfen oder Anschläge (105, 109), die jeweils in den Kammern (C3, C4) befestigt sind, bildet.

**Claims**

1. Device enabling the sterility of a culture medium in an enclosure to be preserved during the withdrawal of samples intended for analysis, comprising, formed in a frame (E; 101–103), a first chamber (C2; C3), known as the withdrawal chamber, in which is slidably mounted a piston (1; 104) provided at one end with means (1b) enabling the withdrawal of a sample to be analysed, and a second chamber (C1; 104), known as the dilution chamber, connected to the first chamber by a passage (12; 112), this second chamber being divided into two zones by stop means (2) against which at least two pistons (3, 4; 106–108) sliding respectively on either side of the stop (2) can come into abutment, the first chamber having at least one outlet orifice for the withdrawn sample, and the second chamber (C1; C4) having at least one inlet orifice (8; 120) for a diluting solvent.

2. Device according to claim 1, characterized in that the piston (1) has a shoulder (1a)) in the zone opposite the withdrawal means.

3. Device according to one of claims 1 and 2, characterized in that sealing means (3a, 4a; 1c, 1d), for example O-rings, are provided between the pistons (3, 4; 1) and the walls of the corresponding chambers (C1; C2).

4. Device according to any one of claims 1 to 3, characterized in that the means enabling the withdrawal of samples consist of a toroidal chamber (1b) formed peripherally on the piston (1).

5. Device according to any one of claims 1 to 4, characterized in that the dilution chamber (C1) is provided with a stop (14) of volume chosen so as to determine the dilution volume delimited by the mutually confronting faces of the pistons (3, 4) and the inner faces of the said chamber (C1).

6. Device according to claim 5, characterized in that the stop (14) is movable and actuated by external means - for example a stepping motor, controlled by electronic computing means - thus enabling the dilution volume, and therefore the dilution coefficient, of the sample to be adjusted.

7. Device according to any one of claims 1 to 6, characterized in that the pistons (1; 3, 4) are actuated by driving means chosen from amongst those of the hydraulic, pneumatic or electromagnetic type.

8. Device according to any one of claims 1 to 7, characterized in that when pneumatic means are used for actuating the pistons, for example via a source of compressed air, air inlet-outlet orifices (5, 6; 11, 10) are provided in the chambers (C1, C2) respectively.

9. Device according to any one of claims 1 to 8, characterized in that the dilution chamber (C1) has an inlet orifice (7) intended for admitting a sterile air/steam mixture intended for cleaning/scavenging the dilution chamber.

10. Device according to any one of claims 1 to 9, characterized in that the driving means are controlled by an automatic cam device (Ac) intended for controlling, according to a preset cycle, inlet solenoid valves (V1, V2, V3, V4, V5, V6, V7) for admitting the different fluids into the withdrawal device.

11. Device according to any one of claims 1 to 9, characterized in that electronic computing means are provided for controlling, according to the desired cycle, valves (V1, V2, V3, V4, V5, V6, V7).

12. Device according to claim 1, characterized in that it comprises two bodies (101, 102) firmly attached to one another and defining a first upper cylindrical chamber, known as the withdrawal chamber, (C3) and a second lower cylindrical chamber, known as the dilution chamber (C4), the first chamber (C3) enclosing a piston (104) which is shaped so as to exhibit a peripheral withdrawal space, whereas the second chamber (C4) encloses three sliding pistons (106, 107, 108).

13. Device according to claim 12, characterized in that the bodies (101, 102) are joined at one end by a plate (103) forming a support for plugs or sealing stops (105, 109) fixed in the chambers (C3, C4) respectively.

FIG. 1

FIG. 2

FIG. 3

FIG. 4

FIG. 5

FIG. 6

EP 0 246 979 B1

FIG. 7

101 110 112 115 104 102 C3 105 118

103

116 119 114 122 120 108 C4 109 117

106 111 107

FIG. 8

101 110 112 115 104 102 C3 105 118

103

116 119 114 122 120 C4 108 109 117

106 111 107

FIG. 9

FIG. 10

EP 0 246 979 B1

FIG. 11A

FIG. 11B

EP 0 246 979 B1

FIG. 12

FIG. 13 A

FIG. 13 B

FIG.14